Europäisches Patentamt

European Patent Office

Office européen des brevets

(11) Publication number: **0 266 640**
**A2**

# EUROPEAN PATENT APPLICATION

(21) Application number: **87115550.3**

(22) Date of filing: **23.10.87**

(51) Int. Cl.⁴: **C07D 211/90** , C07D 473/08 ,
A61K 31/52 , A61K 31/445

(30) Priority: **03.11.86 DE 3637241**

(43) Date of publication of application:
**11.05.88 Bulletin 88/19**

(84) Designated Contracting States:
**AT BE CH DE ES FR GB IT LI NL SE**

(71) Applicant: **Euro-Celtique S.A.**
**122 Boulevard de la Petrusse**
**Luxemburg(LU)**

(72) Inventor: **Dethlefsen, Uwe, Dr.**
**Teichstrasse 28**
**D-5100 Aachen(DE)**
Inventor: **Rackur, Helmut, Dr.**
**Siemensstrasse 2**
**D-6250 Limburg/Lahn(DE)**
Inventor: **Koch, Günther**
**Mundipharma Strasse 2**
**D-6250 Limburg/Lahn(DE)**

(74) Representative: **Eisenführ & Speiser**
**Martinistrasse 24**
**D-2800 Bremen 1(DE)**

(54) A pharmaceutical compound containing nifedipine.

(57) The invention concerns a pharmaceutical compound containing Nifedipine, combined with a physiologically tolerated substance capable of forming a complex with Nifedipine. This increases the solubility of Nifedipine. The complexing substance is preferably a xanthine, especially Theophylline, which gives a surprising synergistic pharmacological combinatory effect. The invention further concerns a pharmaceutical preparation, especially useful for the Nifedipine/Theophylline combination, and a method for producing such preparations.

EP 0 266 640 A2

Fig. 1

## A Pharmaceutical Compound Containing Nifedipine

The invention refers to a pharmaceutical compound containing Nifedipine.

Nifedipine (1,4-dihydro-2,6-dimethyl-4-(2-nitrophenyl)-3,5-pyridine carboxylic acid dimethyl ester) is a well-known calcium antagonist and is used principally in the treatment of coronary heart disease and hypertension. Nifedipine also exhibits a (not very pronounced) broncho-dilatory effect.

The use of Nifedipine is however still restricted by its very low water solubility. This is only of the order of approx. 6 mg/l and renders the desired administration of the required dosage very problematical.

The water solubility of Nifedipine can of course be increased by adding dissolving agents, but this involves strict limitations. A dissolving agent has to be tolerated physiologically, and must not increase the side-effects of the Nifedipine nor contribute any pertinent effects of its own.

The purpose of the invention is therefore to specify a pharmaceutical compound containing Nifedipine, which ensures increased water solubility of the Nifedipine without incurring additional side-effects.

To solve this problem, a pharmaceutical compound containing Nifedipine is prepared with a Nifedipine-complexing substance which is tolerated physiologically.

Specific advantageous embodiments of the combination are set out in the subclaims.

The combination of Nifedipine with a Nifedipine-complexing substance produces a considerable increase in the water-solubility of the Nifedipine. Xanthines are particularly useful for this purpose, especially Theophylline.

Xanthines form complexes with many useful substances, especially sandwich-complexes, whose formation can be attributed to the dipole-dipole-interactions and charge-transfer-interactions of the $\pi$-electron systems between the molecules. This can be exploited, for example, by adding ethylenediamine to increase the solubility of the Theophylline.

The xanthines, particularly Theophylline, also form such complexes with Nifedipine. The combination of Nifedipine with such complexing xanthines, particularly Theophylline, according to the invention, serves to increase the solubility of the Nifedipine.

Molar ratios of 1 : 1 and 2 : 1 for the substances involved are typical for sandwich-complexes, since these complexes - diagramatically speaking - consist of molecules with contiguous molecule surfaces.

However, Nifedipine/xanthine-complexes, especially Nifedipine/Theophylline-complexes do not exhibit a stable equilibrium with molar ratios ranging between 1 : 1 and 2 : 1, with the result that the complex in equilibrium exists only in a very small concentration compared to the individual components and an appreciable increase in the solubility of the Nifedipine is not achieved. This negative effect is enhanced by the high dielectric constant of water.

However, stabilisation is possible if the xanthine, especially the Theophylline, is added in substantial excess of the 1 : 1 ratio. This excess is preferably great enough to give a mole ratio of Theophylline to Nifedipine of between 25 : 1 and 55 : 1 in the pharmaceutical compound.

The use of Theophylline with Nifedipine in in vitro research has been reported by Joubert et al., IRCS Med. Sci. 11,760 (1983), but this was limited to studying the effect of these substances in solution on prepared tracheal muscles. The concentrations which were used (ratio of Theophylline to Nifedipine of the order of 1000 : 1) are inappropriate for pharmaceutical applications. The behaviour in solution of the Nifedipine as a function of the presence of the Theophylline was not investigated.

An increase in the amount of Theophylline to bring it within the ratio with Nifedipine described in the invention initially faced serious pharmacological objections.

Theophylline (1,3-dimethylxanthine) itself shows a pronounced circulatory effect. It is vasodilating and positively inotropic. It was therefore feared that in combinations with Nifedipine, side-effects such as tachycardia, tremor, headache, gastro-intestinal intolerance etc. would be at least cumulative, if not multiplied.

Surprisingly, this fear about cumulative side-effects was not confirmed. Rather, the combination of Nifedipine with Theophylline is actually very well-tolerated.

Astonishingly, although the side-effects are not cumulative, the Nifedipine/Theophylline combination provides synergism with respect to the desired effect. The duration of the effect of the Nifedipine is prolonged; the effect on the patient is greater than the sum of the individual effects of Nifedipine and Theophylline. With the preferred combination of Theophylline and Nifedipine used in the weight ratio of 10 : 1 up to 50 : 1, but particularly in the ratio 30 : 1, there is unexpected optimisation of the effectiveness, which is not achieved with other ratios of the substances.

This synergistic effect is probably a specific property of the combination according to the invention: When administering 10 mg doses of Nifedipine and 300-400 mg doses of Theophylline (as sustained-release tablet) separately to asthma patients, Garty et al (Clin. Pharmacol. Ther., 8/1986, 195) found no effect of the Nifedipine at all.

Since it is only the complex-forming preparation of the invention containing both substances at the same time, which provides a synergistic effect, it may be assumed that a specific pharmaco-dynamic mechanism is involved which is unattainable by the medication described by Garty et al.

The pharmaceutical compound is best formulated in an administrative form where the total content of Nifedipine in each dosage unit is less than that required to develop extensive cardio-vascular effects of the Nifedipine, and where the total content of Theophylline in each dosage unit corresponds to a secretolytic effect largely free of side-effects of the Theophylline alone. In particular, the unit dose of an orally administered tablet, for example, contains approx. 10 mg Nifedipine and 300 mg Theophylline. This dosage unit has a Nifedipine content well beneath the limit at which pertinent cardiovascular effects produced by the Nifedipine occur. The Theophylline content of the dosage unit is still low enough to give a noticeable broncho-dilatory effect, yet the typical side-effects of Theophylline are still largely suppressed, particularly if the Theophylline is not immediately released completely and is only released in complexed form.

Such a formulation surprisingly still shows a strong effect, which in most patients leads to achieving whatever improvement is therapeutically possible. Although the individual concentrations of Nifedipine and Theophylline do not of themselves create such an effect, it is achieved by the combination of the substances. The desired effect must be interpreted as a synergistic effect of the combined substances, since the combination of substances is not only effective over a longer period than the halflife of the components would lead us to expect, but the combined component effect is also greater than the anticipated sum of the individual effects of the components.

In view of this reciprocal synergistic increase in the effect of Nifedipine and Theophylline, it is all the more surprising that no corresponding increase in the side-effects is observed. The overall picture of the side-effects is not at all cumulative; rather the combination can, in most cases, be used until the patients show practically no remaining obstruction of the airways, whithout any disturbing side-effects being observed. The level of side-effects of the combination is roughly the same as the side-effects produced by the individual components in a similar concentration. It is particularly surprising that no tachycardiac effects occur.

The invention thus creates a pharmaceutical compound, which permits the oral administration of a combination of Nifedipine with a water-solubility-enhancing complexing agent, which provides maximum effectiveness in practice, and which, in the process, is largely free of side-effects. The compound can be administered orally most easily in tablet form, but is also suitable for administering in solution.

In addition to Nifedipine and a complex-forming xanthine, or even in place of them, the pharmaceutical compound can contain suitable derivarives thereof, to produce comparable effects. In this case, the weight ratios should be selected such that they likewise correspond to concentrations of the individual components below the side-effect threshold, in order that the total combination develops the desired therapeutic effect due to synergism, and that the desired complexing of the Nifedipine takes place. If instead of Theophylline, a salt-forming xanthine like Aminophylline is used, the composition can e.g. contain same in salt form.

The improved water solubility of the Nifedipine in the compound of the invention can be further optimised by adding a substance which reduces the dielectric constant of the water, especially Polyethyleneglycol (PEG). The PEG, preferably with a mean molar weight between 200 and 6000, produces a considerably increased stability of the Nifedipine/xanthine-complex in aqueous solution. This effect is best achieved when the concentration of PEG, referred to the compound in solution prepared for administration, lies between 0.025 and 0.15 Mol/l.

It was surprisingly found that the pharmaceutical compound need not contain the Nifedipine/xanthine-complex in prepared form. It is sufficient to provide the Nifedipine and the complexing substance together as a simple physical mixture, - for example, to process these separate individual components into a tablet. The complex forms in the desired way upon dissolving the mixture compound in water, both in vitro and in vivo.

The Nifedipine (or a suitable Nifedipine derivative) is, however, preferably contained in the pharmaceutical compound in an oral, solid slow-release formulation, so that the controlled-release effect frequently sought for Nifedipine preparations is achieved.

To obtain this controlled-release effect, the Nifedipine and/or complexing substance can be processed in a micro-granulated form. The solubility of the Nifedipine can, moreover, be positively influenced if the Nifedipine is present in a micronized, partially crystalline or micro-crystalline form.

Advantageous formulations are described e.g. in German Patent 22 24 534, to which reference is expressly made.

An advantageous formulation can accordingly be prepared using a higher aliphatic alcohol, cetostearylalcohol or mixtures thereof, as well as hydrated hydroxyalkylcellulose, the active substances being added to any of these components.

Another advantageous way of formulating the pharmaceutical composition of this invention is by using polyethyleneglycol (PEG) together with hydrated hydroxyalkylcellulose, with the active substances again being added to one or both of said components.

A particular advantage can be achieved if the substance in a dosage unit with approx. 300 mg Theophylline and 10 mg Nifedipine (or convenient multiples) is formulated from a granulated mixture of hydroxyalkylcellulose, polyethyleneglycol and cetostearylalcohol (or a higher aliphatic alcohol), with the Nifedipine being dissolved in the PEG-component. The dosage unit, preferably a tablet, then contains between 20 and 30% by weight of the combined hydroxyalkylcellulose, PEG and cetostearylalcohol components. The ratio of (PEG + cetostearylalcohol) content over hydroxyalkylcellulose content lies preferably in the range (2 - 4) : 1. Said components can initially be produced as two separate granulates, one containing the Theophylline, the other the Nifedipine; the granulates are thereafter mixed and the mixture is pressed into tablets.

A tablet produced in this way exhibits an unexpected, but very beneficial effect. In the aqueous medium, the tablet does not dissolve immediately into the primary granulate with the Theophylline being released in one surge, which in view of the high concentrations of Theophylline besides Nifedipine, would possibly be detrimental.

Rather the tablet matrix is sustained for several hours and only dissolves then into the primary granulate. After being administered, the Theophylline/Nifedipine complex is slowly released in a benificial manner, with the components producing the desired synergistic effect without appreciable harmful side-effects. It is assumed that this surprising effect is based on the complexing relationship between the Theophylline and· the Nifedipine being already effective upon release from the solid compound.

A unit dosage tablet formulation of the preparation according to the invention, which has shown especially great usefulness in praxi, is produced according to the following example:

## EXAMPLE

1. Preparation of granulated Theophylline component:
600 g of Theophylline (dry) and 37,5 g hydroxyalkylcellulose were mixed (dry), then granulated with approx. 3 % polyvinylpyrrolidon (Kollidon 25) (solution in water); the granulated mixture was then dried.

In a separate vessel, 60 g cetostearyl alcohol and 75 g polyethyleneglycol PEG 6000 were melted and the melt was then added to the dried granules. After cooling, the granules were processed to a final granule size of approx. 1 mm and then mixed with 7.5 g Mg-stearate and 7.5 g Talcum.

2. Preparation of granulated Nifedipine component:

122 g lactose, 24 g Primojel and 20 g hydroxyalkylcellulose were mixed (dry), then granulated with ethanol and dried.

Separately, 20 g Nifedipine were dissolved in a melt of 20 g cetostearylalcohol and 50 g PEG 1500; the solution was then added to the dry lactose granules. After cooling, the granules were processed to final granule size of approx. 1 mm and mixed with 4 g Mg-stearate and 8 g Talcum.

3. The granules obtained by the above steps 1. and 2. were mixed and the combined granules were then pressed into oblong tablets of 545 mg average tablet weight.

The tablets were coated with a light-protective coating (lacquer).

Below, the unexpected synergistic effect of the pharmaceutical compound of the invention is further elucidated by results of treatment.

These were observed in a clinical test on asthmatic patients, when Nifedipine alone, Theophylline alone or the combination of the invention, respectively, were used.

The result are shown in Figures 1 to 3.

Fig. 1 shows the change in the airway resistance (RAW) of asthmatic patients after treatment with Nifedipine alone, with Theophylline alone or with the Nifedipine/Theophylline combination, respectively.

Fig. 2 shows the change in the 1-second-capacity $FEV_1$, measured on asthmatic patients after treatment with Nifedipine alone, Theophylline aloneor with the Nifedipine/Theophylline combination, respectively;

Fig. 3 shows the change in mean-value pulse-rate of the patients during the treatment as in Figs. 1 and 2.

In a controlled triple-cross-over study, in a body phlethysmographic test of the lung function, the effectiveness of and tolerance to the individual substances compared to the combination were investigated.

The investigation was carried out on three in-patients with chronic-obstructive airway diseases. The study only concerned patients whose initial airway resistance was in the range from 6 to 12 cm $H_2O/1/s$, and whose 1-second-capacity $FEV_1$ after inhalation of two draws each of 200 microgram Fenoterol showed an increase of at least 15 %.

The study only involved suitable patients who did not require any other form of treatment. The associated medication with dosing aerosols was reduced to a minimum and noted in a special test report. It was ensured that no aerosol was used at least eight hours prior to each lung function investigation.

Patients with secondary diseases, which could have jeopardized an evaluation of the treatment owing to their progressive nature, were not included in this study.

The patients were treated either with Nifedipine in 10 mg doses, with Theophylline in 300 mg doses or with the Nifedipine/Theophylline combination of the invention in the ratio 10 mg : 300 mg per dosage unit. Either 10 mg Nifedipine, or 300 mg Theophylline or the combination with 10 mg Nifedipine and 300 mg Theophylline were administered at 08.00 hours and 20.00 hours. The medication sequence was randomised, with a wash-out-phase of two days after each medication.

It was ensured that all the patients had received no xanthine 24 hours before the controlled investigation (lung function test and determination of reversibility).

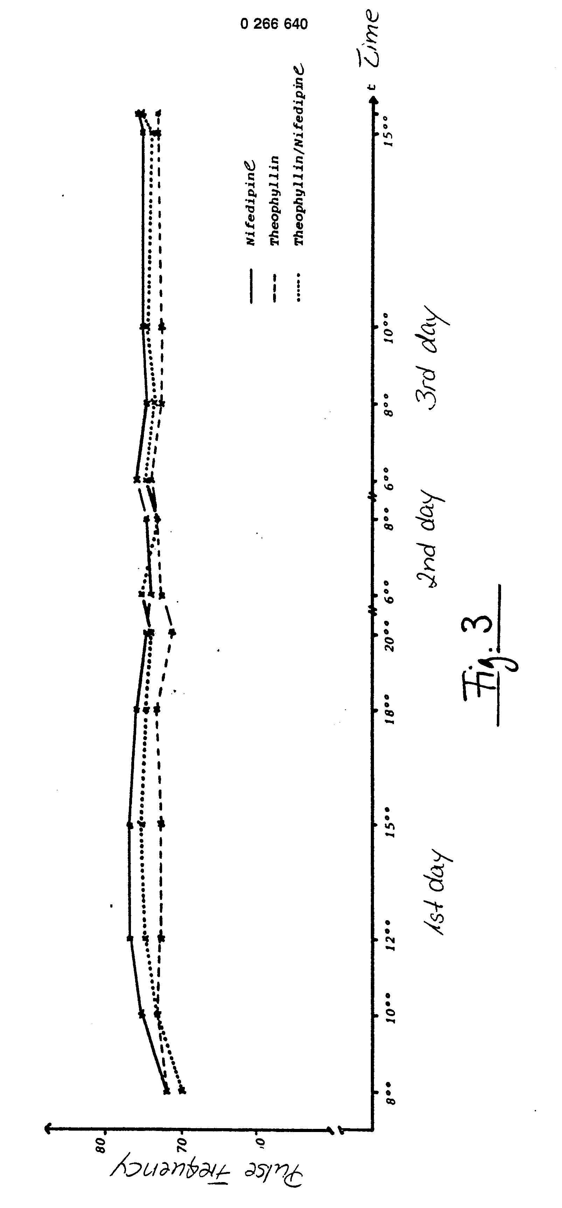

The lung function (RAW and $FEV_1$) and the pulse of the patients were measured on the first and third day always at the same time of day, as shown in Fig. 3. On the third day of the investigation, after the final measurement of lung function, the reversibility was again determined, 15 minutes after inhalation of 400 microgram Fenoterol.

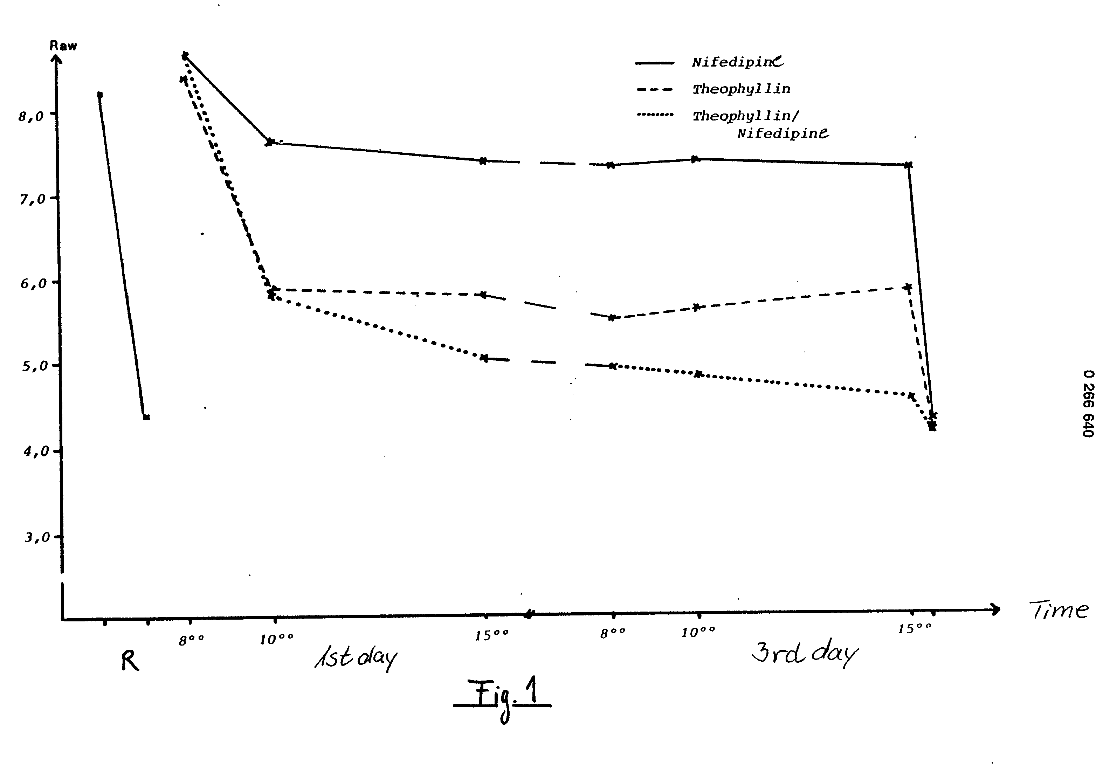

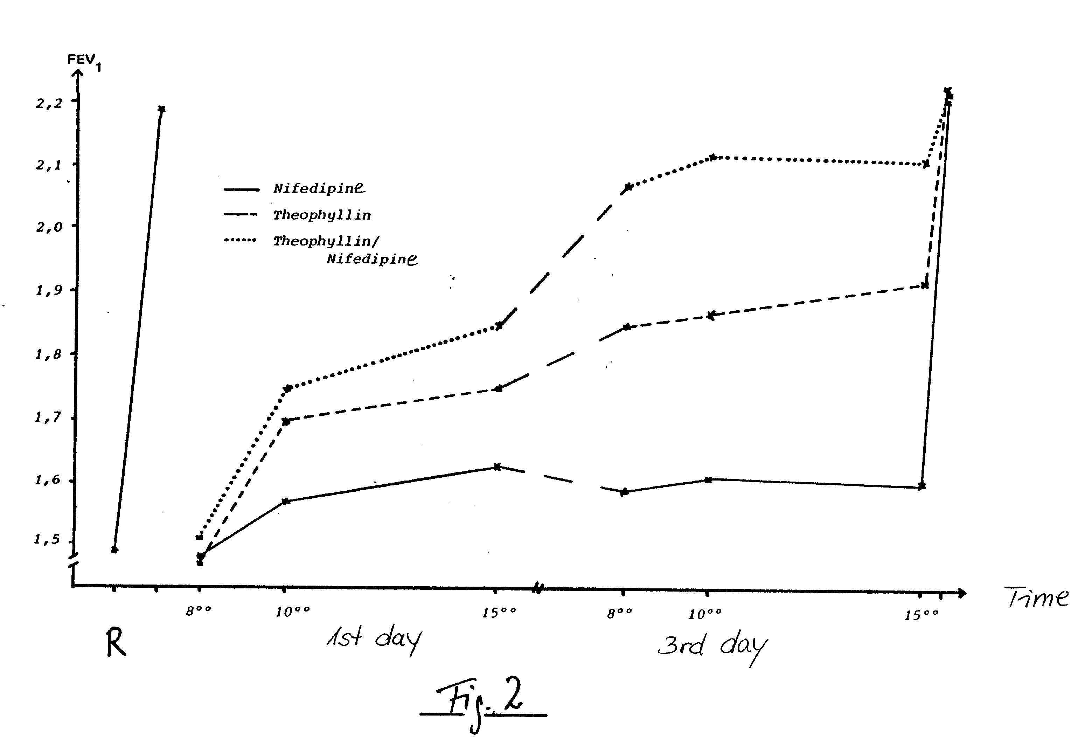

The determination of the output airway resistance and the measurement of reversibility at the beginning of the investigation showed that the patients had a medium grade obstruction, which decreased after inhalation of 400 microgram Fenoterol, corresponding to a reduction of 50 % (referred to the initial value). This preliminary determination of reversibility is indicated in Figs. 1 and 2 with R.

The change of airway resistance (RAW) shown in Fig. 1 (mean values of the airway resistances of the three patients) clearly shows that the effect of the combination is superior to the effects of the individual substances in a statistically highly significant way.

In particular, it is seen that at the end of the treatment period, a considerably greater approximation to the therapeutically achievable improved state was obtained when the combination was administered. This is illustrated by the remaining reversibility, measured on the third day after renewed inhalation of 400 microgram of Fenoterol (15.00 hours-value).

Figure 2 shows the change in the 1-second-capacity $FEV_1$, again using the mean values of the three patients. Here too, before commencing the actual series of tests, the reversibility (R) was measured before and after inhalation of 400 microgram Fenoterol. After the tests had ended, the remaining reversibility was measured in the same way (at 15.00 hours on the third day).

At the start of the investigation, the patient showed a $FEV_1$ mean-value of ca. 1,5 l, which rose by 45 % to ca. 2.2 l after inhalation of 400 microgram Fenoterol (reversibility). Lung function of the patients treated with the combination of the invention was statistically highly significantly better on the third day, compared to that of the patients treated with the individual substances. Determination of the remaining reversibility on the third day showed that both Nifedipine treatment alone and Theophylline treatment alone still left a considerable further improvement by inhalation of the dosing aerosol, whereas on the third day of treatment with the combination of the invention, reversibility only corresponded on average to a practically negligible increase in $FEV_1$ of ca. 100 ml. Fig. 2 illustrates that, compared to the situation achieved by the combination of the invention, scarcely any improvement was achieved; these patients showed practically no longer any obstruction of the airway.

As a check particularly with respect to side-effects, the mean-values of the patient's pulse-rates were recorded throughout the whole duration of the treatment period (Fig. 3). As this Figure clearly shows, the side-effects during treatment with the combination of the invention were not appreciably greater, and in part even smaller, than during treatment with corresponding doses of the individual substances. The mean-values for blood pressure recorded in parallel (not shown) showed in a wholly analogous way the absence of side-effects due to the combination, which could affect blood pressure.

In the evaluation, it can be said that the well-known effects of Theophylline seen in the investigation are also seen in the treatment with this substance alone. The anticipated bronchodilatory effect was confirmed for Nifedipine, which effect turned out to be quite different in different patients. Both treatments with the individual substances thus fully agree with the effects attributed to them in the literature. In this context, it is shown that the test set up and the evaluation of the data are meaningful in practice.

The distinct superiority of the combination compared to the individual substances is shown by comparing the lung function measurements. The clinically significant improvement in lung function produced by the combination is reliably established statistically. In this study, the pulse showed no clinically relevant irregularities; nor were any side effects observed for blood pressure.

Thus, the combination of substances according to of the invention is shown to be synergistically highly effective in clinical tests in vivo. Hence the combination of the substances of the invention can be used to produce a pharmaceutical preparation for treatment with a combination of Nifedipine/Theophylline in the simplest way. It achieves a significant reduction in the symptoms of the complaint and makes a high degree of bronchodilation possible without causing appreciable side-effects.

The unit dosage formulation described in the above example is useful for pharmaceutical preparations other than the special Nifedipine/Theophylline preparation as illustrated. It can be used for any preparation containing Nifedipine and a complexing substance, but its usefulness is not even restricted to the production of Nifedipine preparations. Actually, this method of formulating a pharmaceutically active substance or ingredient is useful for sustained-release preparations of various active substances. It is not restricted to active substances which do not the dissolve easily in physiological fluids, although it is highly useful for such substances, which otherwise are difficult to administrate efficiently.

The formulation described in the example is only one special embodiment. The minimum of features necessary for providing such a formulation according to this invention is disclosed in appended claim 21. Further, more specific embodiments are described by claims 22 through 34, which will easily be seen to include the features of the special formulation of the above-described example. A repetition of these further embodiments in this description is therefore considered unnecessary. For other purposes than the formulation of Nifedipine/Theophylline, a suitable feature combination of claims 21 through 34 will be chosen according to the requirements, especially the desired release duration of the active ingredient(s) to be formulated, their solubility, degree of micronization,physiological effect, bioavailability a.s.o.

## Claims

1. A pharmaceutical compound containing Nifedipine, characterized by the compound also containing a physiologically tolerated substance capable of forming a complex with Nifedipine.

2. A compound according to Claim 1, characterized by the molar concentration of the complexing substance in the compound being greater than that of the Nifedipine.

3. A compound according to Claim 1 or 2, characterized by the compound containing the complexing substance and the Nifedipine in a molar ratio of between 25 : 1 and 55 : 1.

4. a compound according to one of Claims 1 to 3, characterized by the complexing substance being a xanthine.

5. A compound according to Claim 4, characterized by the complexing substance being Theophylline.

6. A compound according to Claim 5, in the form of a pharmaceutical preparation, characterized by the total concentration of Nifedipine in each dosage unit being less than that required to develop appreciable cardio-vascular effects of the Nifedipine, and by the total concentration of Theophylline in the dosage unit being chosen in accordance with a bronchodilatory effect, still largely free of side-effects, of the Theophylline alone.

7. A compound according to Claim 5 or 6, characterized by concentrations of Nifedipine and Theophylline in the weight ratio of 1 : 10 up to 1 : 50, preferably of 1 : 30, and particularly preferred with approx. 10 mg Nifedipine and 300 mg Theophylline in each dosage unit.

8. A compound according to one of the Claims 1 to 7, characterized by the content, in addition if necessary, of a derivative of Theophylline like Aminophylline or a salt of the derivative, and/or of Nifedipine.

9. A compound according to one of the Claims 1 to 8, characterized by the content of a substance which reduces the dielectric constant of water.

10. A compound according to one of the Claims 1 to 9, characterized by a content of Polyalkyleneglycol, especially Polyethyleneglycol (PEG).

11. A compound according to Claim 10, characterized by the PEG having a mean molar weight between 100 and 10000, preferably between 200 and 6000.

12. A compound according to Claim 10 or 11, characterized by the content of PEG being of the order of between 0.025 and 0.15 Mol/l, referred to the compound in solution ready for administration.

13. A compound according to one of Claims 1 to 12, characterized by the compound containing the Nifedipine and the complexing substance as well as further additional agents, if required, as a physical mixture.

14. A compound according to one of Claims 1 to 13, formulated for oral, parenteral or transdermal administration.

15. A compound according to Claim 14 for oral administration, particularly in tablet form, containing the solid active substances.

16. A compound according to Claim 14 or 15, characterized by the compound containing the Nifedipine and/or the complexing substance in granulated form, for controlled slow release.

17. A compound according to one of Claims 14 to 16, characterized by the compound containing the Nifedipine and/or the complexing substance in micronized, partly crystalline or micro-crystalline form.

18. A compound according to one of Claim 14 to 17, characterized by the dosage unit
a) containing substantially 300 mg Theophylline and 10 mg Nifedipine, and
b) containing between 20 and 30 % by weight (referred to the total weight of the dosage unit) of a granulated mixture of polyethyleneglycol, hydroxyalkylcellulose and a higher aliphatic alcohol or cetostearylalcohol,
c) at least the Nifedipine being contained in the granules of the mixture.

19. A compound according to Claim 18, characterized by the Nifedipine and the Theophylline being contained in PEG-components of the mixture.

20. A compound according to Claim 20, characterized by the Nifedipine being at least partially dissolved in the PEG-component.

21. A method for producing a solid pharmaceutical preparation, characterized in that
a first granulate containing hydrated alkylcellulose is prepared;
a substantially water-insoluble waxy substance is melted or otherwise made flowable and a pharmaceutically active ingredient is suspended in the flowable waxy substance, which thereafter is allowed to solidify;
a second granulate is prepared containing the waxy substance together with the active ingredient;
and both granulates are thereafter combined and formed into the solid preparation, if desired together with customary additives.

22. Method according to claim 21, characterized in that the waxy substance comprises cetostearylalcohol, one or more higher aliphatic alcohols, especially $C_8$ to $C_{36}$ alcohols, polyalkyleneglycol, especially polyethyleneglykol and/or sorbitan fatty acid ester.

23. Method according to claim 21 or 22, characterized in that one granulate comprises a pharmaceutical bursting agent providing fast disintegration of the primary granulate into individual granulate particles.

24. Method according to any one of claims 21 through 23, characterized in that the preparation further contains water-soluble alkylcellulose of low viscosity.

25. Method according to any one of claims 21 through 24, characterized in that the dry components of the first granulate are mixed and granulated under addition of substantially non-aqueous alcohol, preferably 96%-ethanol.

26. Method according to any one of claims 21 through 25, characterized in that the pharmaceutically active ingredient is at least partly dissolved in the waxy substance prior to granulation of the second granulate.

27. Method according to any one of claims 21 through 26, characterized in that both granulates contain alkylcellulose and polyalkyleneclycol and further contain cetostearylalcohol, higher aliphatic alcohol and/or sorbitan fatty acid ester.

28. Method according to any one of claims 21 through 27, characterized in that both the first and the second granulate contain an active ingredient, the first granulate containing a different active ingredient than the second granulate.

29. Method according to any one of claims 21 through 28, characterized in that one of the active ingredients, especially the active ingredient contained in the second granulate, has low solubility in physiological fluids, especially of the order of 10mg or less ingredient in 100ml fluid.

30. Method according to any one of claims 21 through 29, characterized in that one of the granulates also contains lactose.

31. Method according to any one of claims 21 through 30,
characterized in that the polyalkyleneglycol, especially polyethyleneclycol, has an average molecular weight between 200 and 10000, preferably between 500 and 6000 and especially between 1500 and 3000.

32. Method according to any one of claims 21 through 30,
characterized in that the first granulate contains polyethyleneclycol with an average molecular weight of approximately 6000 and that the second granulate contains polyethyleneclycol with an average molecular weight of approximately 1500.

33. A method according to any one of the claims 21 through 32,
characterized in that the solid pharmaceutical preparation contains between 20 and 30% by weight of the combined alkylcellulose and waxy substance components.

34. Method according to any one of claims 21 through 33,
characterized in that 2 to 4 parts of waxy substance are used for every part of alkylcellulose or hydroxyalkylcellulose.

35. Pharmaceutical preparation, prepared according to any one of claims 21 through 34.

Fig. 1

Fig. 2

0 266 640

Fig. 3